# EUROPÄISCHE PATENTSCHRIFT

(11) **EP 2 261 636 B1**
(45) Veröffentlichungstag und Bekanntmachung des Hinweises auf die Patenterteilung: **11.09.2013**
(21) Anmeldenummer: 10002623.6
(22) Anmeldetag: 12.03.2010
(51) Int. Cl.: G01N 19/04, G01N 3/00, G01N 33/32

(54) **Gitterschnitt-Prüfgerät**
Cross-cut test device
Appareil de vérification à quadrillage

(30) Priorität: 25.03.2009 DE 102009014680
(43) Veröffentlichungstag der Anmeldung: 15.12.2010
(73) Patentinhaber: Schwickert, Franz, 56414 Oberahr (DE)
(72) Erfinder: Schwickert, Franz, 56414 Oberahr (DE)
(74) Vertreter: Hannke, Christian

(56) Entgegenhaltungen:
- EP-A1- 0 049 090
- DD-A1- 155 554
- DE-A1- 1 956 098
- US-A- 3 389 463

## Beschreibung

Die Erfindung bezieht sich auf ein Gitterschnitt-Prüfgerät mit
- einem gegen eine Beschichtung andrückbaren Messerkopf mit mehreren Schneidklingen und
- einem bevorzugt drehbar daran angebrachten Handgriff.

Um die Haftung einer Beschichtung auf einem Substrat zu testen, etwa von Farbe oder Verchromung auf einer Metalloberfläche, wird seit vielen Jahren der Gitterschnitt verwendet. Dabei handelt es sich um ein Raster aus einander rechtwinklig kreuzenden Schnittlinien, die, in einem begrenzten Bereich, die Beschichtung durchtrennen, ohne das Substrat wesentlich zu verändern. So entstehen auf dem Substrat kleine Beschichtungs-Quadrate mit einer Kantenlänge von 1, 2 oder 3 mm (Norm) oder 1,5 mm (Daimler-Benz), von deren Vorhandensein bzw. vollständigem oder teilweisem Abplatzen mit ausreichender Sicherheit auf die Haftung der gesamten Beschichtung auf dem Substrat geschlossen werden kann. So werden etwa im Fahrzeugbau Stichproben vorgenommen, um sich zu vergewissern, dass auf Stoßstangen, Außenspiegeln oder dergleichen die Verchromung oder der Lack hinlänglich dauerhaft aufgebracht sind und sich nicht Mängel in der Qualität der Beschichtung einschleichen. Im Einzelfall kann sogar jedes Werkstück an einer nach dem Einbau nicht mehr sichtbaren Stelle geprüft werden oder für jedes Werkstück ein zu prüfendes Bezugsstück angefertigt werden.

Es gibt aufwendige Geräte zum maßgenauen Einbringen des Gitterschnitts, beispielsweise beschrieben in der Druckschrift DE 202 16 786 U1, aber meist wird ein kleines Handgerät verwendet, mit dem auf einer Werkstückprobe von Hand hinlänglich genaue Schnitte durch überkreuzende Führung des Geräts eingebracht werden.

Ein solches Gerät ist das Gitterschnitt-Prüfgerät Modell 295 der Firma Erichsen GmbH & Co. KG, Hemer, Deutschland. In diesem Gerät sind scheibenförmige Schneiden in einem gegenseitigen Abstand angeordnet, der einer gängigen Norm entspricht. Die Schneiden sitzen fest in einem Kopf, der drehbar an einem Handgriff angebracht ist, um die Handhabung des Gerätes zu vereinfachen und für einen gleichmäßigen Eingriff zu sorgen. Außerdem sind die Schneiden zu einem zusammenhängenden Körper zusammengefasst, um ein gleichmäßiges Eindringen aller Schneiden in die Beschichtung zu gewährleisten. Durch Drehen dieses Schneidenkörpers können neue Schneiden in Einsatz gebracht werden, wenn die früheren Schneiden schadhaft geworden sein sollten. So stehen bis zu vier Sätze von Schneiden pro Körper zur Verfügung.

Ferner gehören Dokumente DD 155 554 A1 und DE 1956 098 A1 zum Stand der Technik.

Der Hauptnachteil der bekannten Einrichtung liegt, neben den erheblichen Kosten für den hochgenau zu fertigenden und zu schleifenden Schneidenkörper, in der Tatsche, dass in den meisten Fällen das Substrat nicht hinlänglich eben ist, so dass trotz genauester Fertigung und Handhabung benachbarte Gitterlinien die Beschichtung nicht ganz durchdringen oder in das Substrat unter Bildung eines Grates einschneiden, so dass die Messwerte verfälscht sind. Sollte ferner eine Schneide des Schneidenkörpers schadhaft sein, ist der gesamte Schneidenkörper zu drehen oder auszutauschen, auch wenn alle anderen Schneiden noch intakt sein sollten. Zwar wäre es möglich, anstelle des Schneidenkörpers einzeln eingebaute Schneiden vorzusehen, doch würde der genaue Einbau dieser Schneiden ebenfalls sehr aufwendig sein; im Werkstattbetrieb ist es erheblich einfacher, einen einstückigen Scheidenkörper einfach gegen einen anderen auszutauschen, als durch Einbau und Vermessen einer Einzelschneide das Gerät wieder brauchbar zu machen. Außerdem kann der Schneidenkörper einfach gegen einen mit einem anderen Schneidenabstand ausgetauscht werden, wenn ein anderes Gitterraster verwendet werden sollte.

Obwohl eine Vereinfachung des recht einfachen Geräts kaum mehr denkbar ist, ist es Aufgabe der Erfindung, dennoch für eine Verbesserung und besonders Verbilligung und, insgesamt, für ein anderes, neuartiges Schneidgerät zu sorgen.

Diese Aufgabe wird dadurch gelöst, dass
- die Schneidklingen einzeln gegen die Beschichtung hin mit mindestens einer Feder abgefedert sind, und
- beiderseits des Messerkopfes ein Führungskörper angeordnet ist, der auf der Beschichtung aufsitzt und den Messerkopf in einem bestimmten Abstand von der Beschichtung führt.

Zunächst werden anstelle eines Schneidenkörpers mit mehreren Klingen Einzelklingen eingeführt, wie sie oben schon als nachteilig beschrieben wurden. Diese Einzelklingen müssen aber erfindungsgemäß nicht hochgenau angebracht werden, sondern sind federbelastet. Es wird also nicht die geometrische Lage einer Klinge eingestellt, sondern die Kraft, mit der sie gegen die zu prüfende Oberfläche gedrückt wird. Hat jede Einzelklinge nur ungefähr dieselbe Lage, ist aber mit der gleichen Federkraft belastet, dann ergibt sich sogar ein deutlich verbesserter Gitterschnitt als mit dem bekannten Schneidenkörper, wenn etwa das Substrat nicht genau eben ist, weil nicht die geometrische Lage der Schneide die Tiefe des Gitterschnitts bestimmt, sondern die die Schneide einzeln belastende Kraft. Es muss das Gerät nicht "mit Gefühl" über eine zu prüfende Oberfläche geführt werden, sondern es muss nur hinlänglich gegen diese angedrückt werden. Dazu sind jedoch die Führungskörper Voraussetzung, die erfindungsgemäß beiderseits der Schneiden des Geräts angebracht sind. Ohne diese wäre das Gerät, nur mit den abgefederten Schneiden ausgestattet, nahezu unbrauchbar.

Die Führungskörper können beispielsweise als Gleitsteine ausgebildet sein, die vom erfindungsgemäßen Gerät gegen die zu prüfende Fläche angedrückt werden und das Gerät führen. Bevorzugt ist jedoch, dass jeder der Führungskörper von einer Führungsrolle gebildet ist, die drehbar beiderseits des Geräts angebracht ist. Diese Führungsrolle erleichtert die Bewegung des Gerätes über die zu prüfende Fläche und schont diese.

Die Schneidklingen selbst können als Messerklingen ausgebildet sein, die jeweils von einer Feder belastet werden. Es ist aber bevorzugt, dass jede der Schneidklingen von einem drehbar im Messerkopf sitzenden, angespitzten Stift, insbesondere Hartmetallstift, gebildet ist. Dieser Hartmetallstift besteht aus einem äußerst harten Material und weist, wenn überhaupt, nur eine geringe Abnutzung auf. Diese Stifte oder Hartmetallstifte sind gegenüber dem Messerkopf federnd beweglich und sind verhältnismäßig preisgünstig herzustellen. Sie können, je nach zu prüfender Beschichtung, sehr unterschiedliche Spitzenwinkel aufweisen, doch der bevorzugte Spitzenwinkel liegt bei 30°. Diese Spitzen schneiden oder kratzen in die Beschichtung, wobei sie senkrecht zu dieser stehen, so dass aus der Bewegung des Geräts über die Beschichtung keine oder nur eine geringe Kraft auf diese Stifte resultiert. Ihre Lage zu dieser Beschichtung ergibt sich alleine aus der Position der Führungskörper und der Federkraft. Die Spitzen können alle von einer einzigen Feder belastet sein, doch ist bevorzugt eine Feder pro Spitze vorgesehen. Diese Federn können nun unterschiedlich eingestellt sein, so dass z.B. das Gerät auch auf kugeligen Oberflächen eingesetzt werden kann, ohne baulich irgendwie verändert zu werden, sondern nur durch Einstellung der Federkraft. Die Federn geben außerdem bei unterschiedlicher Dicke der Beschichtung oder bei leicht unebener Fläche dem Widerstand des Substrats nach, vorausgesetzt, dieses Substrat ist härter als die Beschichtung. Die Messergebnisse sind daher bei solchen Flächen in der Regel erheblich genauer, als sie es bei fest eingestellten, unnachgiebigen Schneiden wären.

Eine besonders bevorzugte Ausgestaltung der Erfindung liegt darin, dass um jeden Hartmetallstift herum eine Spiral-Druckfeder angeordnet ist, die sich einerseits auf einem Bund oder dergleichen des Hartmetallstifts und andererseits auf einem Auflager des Messerkopfes abstützt. Diese Spiralfedern sind mit ausreichender Genauigkeit und wesentlich billiger herstellbar, verglichen etwa mit Blattfedern.

Die Hartmetallstifte können etwa den Querschnitt eines regelmäßigen Vielecks aufweisen. So kann die Drehlage des Stiftes mittels z.B. einer Rast einfach eingestellt werden. Da aber die Abnutzung der Stifte gering ist, wird bevorzugt, dass der Hartmetallstift rund ist. So stellt sich der Stift von selbst je nach seiner Abnutzung ein, sollte einmal eine solche auftreten.

Eine bevorzugte Ausgestaltung der Erfindung besteht darin, dass der Hartmetallstift in einer Bohrung des Messerkopfes geführt ist, auf deren Einlass der Bund als Endanschlag aufsitzt. So können die Stifte nicht versehentlich aus dem Gerät herausfallen. Natürlich wird beim Prüfen einer Beschichtung dieser Endanschlag nicht erreicht.

Dabei ist es besonders vorteilhaft, wenn der Abstand des Auflagers vom Einlass der Bohrung einstellbar ist. So kann durch Einstellen des Abstands die Federkraft verändert werden, sei es, um das Gerät an eine jeweilige Beschichtung anzupassen, sei es, um einzelne Stifte zur Prüfung einer unebenen Fläche unterschiedlich einzustellen.

Dabei besteht eine bevorzugte Ausgestaltung darin, dass das Auflager von einer Schraubenspindel, bevorzugt einer Madenschraube, gebildet ist, die zur Bohrung koaxial ist. Durch Ein- und Ausschrauben der Spindel oder Madenschraube kann die Federkraft mühelos mittels eines Schraubenziehers oder Schraubenschlüssels eingestellt werden.

Wenn die Federn mit hinlänglicher Genauigkeit gefertigt werden, dann ist es möglich, dass die Schraubenspindel und/oder die benachbarte Seite des Messerkopfes markiert ist bzw. sind, um die Kraft der Feder einzustellen. So ist es möglich, in einer laufenden Serienfertigung das Gerät einfach und mühelos auf vorher ermittelte Werte einzustellen bzw. umzustellen.

Bei dem eingangs genannten, bekannten Gerät sitzen die Schneiden dicht nebeneinander. Nach einer Ausgestaltung der Erfindung ist es dagegen von Vorteil, dass mindestens ein Teil der Hartmetallstifte zueinander in Schnittrichtung versetzt angeordnet ist. Durch diese Versetzung ist es möglich, Stifte und Federn zu verwenden, deren Durchmesser wesentlich größer ist als der seitliche Abstand der Schnittlinien zueinander. Die teuere und empfindliche Miniaturfertigung wird dadurch vermieden. Bevorzugt sind jeweils drei gestaffelte Stifte, deren Abstand quer zur Schnittrichtung nur einen Millimeter beträgt, deren Durchmesser aber wesentlich größer ist, ohne dass sie einander beeinträchtigen.

Um mit ein und demselben Gerät unterschiedliche Raster herzustellen, ist es von Vorteil, dass einzelne Hartmetallstifte unwirksam gemacht oder ausgebaut werden können. Zum Beispiel ist es möglich, jeden zweiten oder dritten Stift anzuheben und zu verrasten, um eine 2 mm- oder 3 mm-Teilung zu erhalten, und zwar mit einem Gerät, das für eine 1 mm- Teilung eingerichtet ist. Zudem ist es möglich, die relative Anordnung der Stifte zueinander in verschiedenen Formen vorzunehmen, um unter geringem Aufwand von einander beispielsweise 1 mm, 2 mm und /oder 3 mm beabstandete Gitterschnittlinien zu erzeugen. Vorzugsweise erfolgt die geometrische Anordnung mehrerer Bohrungen im Messerkopf zumindest teilweise senkrecht und/oder schräg zur Schnittrichtung, besonders bevorzugt hintereinander gestaffelt; sie kann jedoch auch andere zweckmäßige Formen annehmen.

Das erfindungsgemäße Gerät ist demnach billiger als das eingangs genannte, bekannte Gerät, ist ohne Erfahrung handhabbar, ist vielfältiger und insbesondere auch bei unebenen oder gekrümmten Flächen noch zu einer aussagekräftigen Messung verwendbar.

In der schematischen Zeichnung ist als Ausführungsbeispiel ein erfindungsgemäßes Gitterschnitt-Prüfgerät gezeigt. Es zeigen:
- Fig. 1: einen Schnitt in Seitenansicht des Gerätes, längs Linie A-A in Fig. 2,
- Fig. 2: einen Schnitt des Gerätes, von unten her gesehen, längs Linie B-B in Fig. 1, und

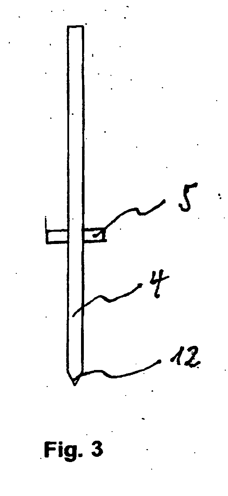

- Fig. 3: einen Hartmetallstift in vergrößertem Maßstab;
- Fig. 4: einen Schnitt in Seitenansicht des Gerätes gemäß einer weiteren Ausführungs- form der Erfindung und
- Fig. 5: einen Schnitt des Gerätes, von oben und von unten her gesehen gemäß der weiteren Ausführungsform der Erfindung.

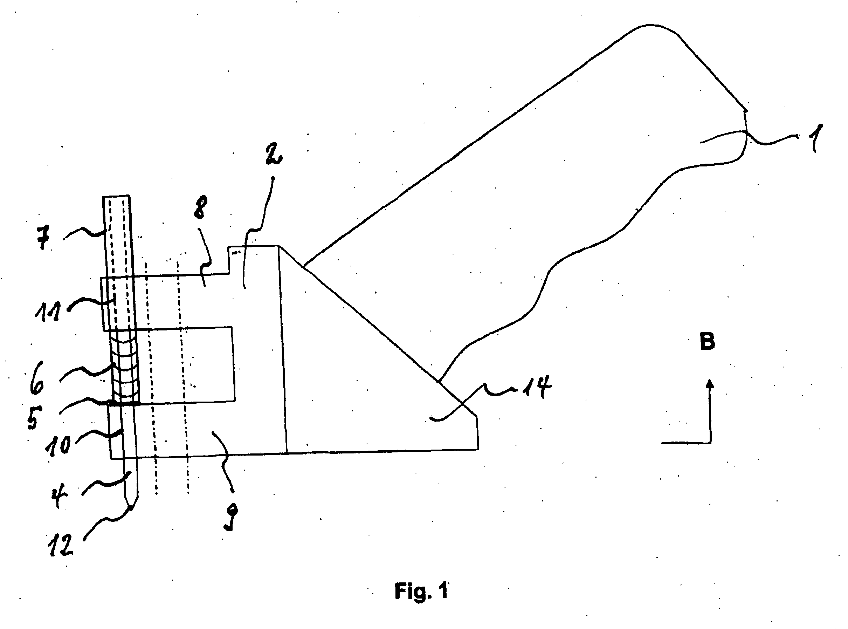

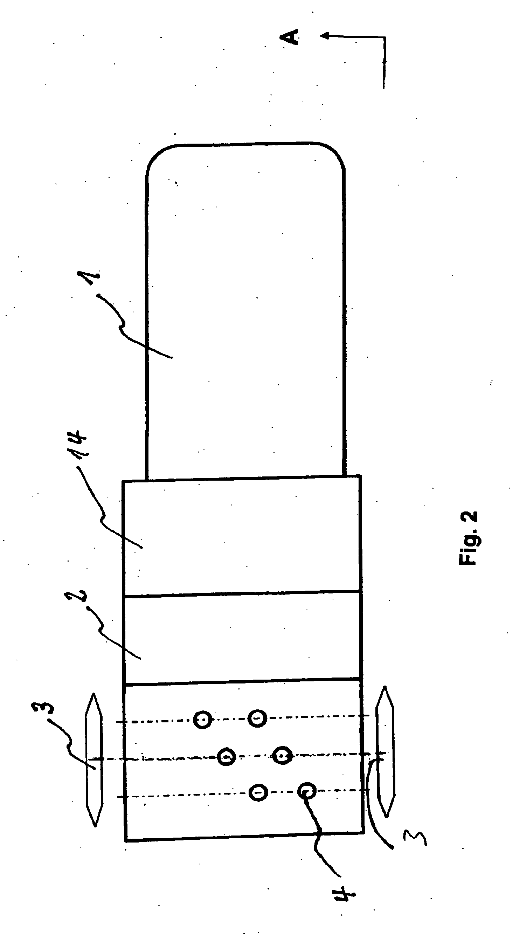

Die Figuren 1 und 2 sind etwa maßstäblich dargestellt.

Der Aufbau des Geräts ist aus Fig. 1 ersichtlich. Ein Handgriff 1 ist fest an einem Kopfteil 14 angebracht. Das Kopfteil 14 ist schwenkbar (siehe Pfeil C in Fig. 2) an einem Kopf 2 befestigt. Dieser Kopf 2 wird beim Gebrauch in Richtung der Schnittlinien A-A und B-B über eine hier nicht dargestellte, zu prüfende Oberfläche geführt, auf der Führungsräder 3 (in Fig. 1 weggelassen) abrollen. Die Achse dieser Führungsräder 3 ist in Fig. 2 mit 13 bezeichnet. Beide Führungsräder können durch diese Achse 13 fest miteinander verbunden sein, um ein geradliniges Abrollen des Gerätes auf einer Oberfläche sicherzustellen. Die zu prüfende Oberfläche ist parallel zur Schnittlinie B-B in Fig. 1.

Der Handgriff 1 steht schräg zur genannten, zu prüfenden Oberfläche und ist am Kopfteil 14 befestigt, das seinerseits um eine Achse, die parallel zur Schnittlinie B-B in Bewegungsrichtung des Geräts verläuft, am Kopf 2 schwenkbar angebracht ist, um der den Griff haltenden Hand eine je nach Lage geeignete Position zu ermöglichen.

Der Kopf 2 weist eine etwa würfelfömige Außenfläche auf, die von vorne her, auf die Bewegungsrichtung bezogen, eingeschnitten ist. Dieser Einschnitt unterteilt den Kopf 2 in ein Oberteil 8 und ein Unterteil 9, die nahe dem Kopfteil 14 durch ein massives Stück verbunden sind. Das Oberteil 8 und das Unterteil 9 werden durch eine Reihe von Gewindebohrungen 11 und dazu koaxialen Durchgangsbohrungen 10 durchsetzt. Diese paarweise koaxialen Bohrungen, die Durchgangsbohrungen 10 und die Gewindebohrungen 11, verlaufen insgesamt zur zu prüfenden Oberfläche senkrecht. Dabei ist jeweils die untere Bohrung 10 als sauber geriebene, durchgehende Zylinderbohrung ausgebildet, während die jeweils zur unteren Bohrung 10 koaxiale obere Gewindebohrung 11 als durchgehende Gewindebohrung ausgebildet ist, deren kleinster Innendurchmesser größer ist als der Durchmesser der Zylinderbohrung 10. Im dargestellten Beispiel sind insgesamt je sechs Bohrungen 10 und je sechs Gewindebohrungen 11 vorgesehen, die jeweils zu dreien gestaffelt sind, wie aus Fig. 2 hervorgeht, auf die ausdrücklich hingewiesen wird.

Ein geschlichteter, bevorzugt polierter Hartmetallstift 4 mit rundem Querschnitt sitzt in jeder Bohrung 10, und zwar mit nur ganz leichter Spielpassung, so dass der Hartmetallstift 4 noch mit Leichtigkeit in der geriebenen Bohrung 10 beweglich ist, sich aber in keiner Weise schräg zur Bewegungsrichtung des Geräts (Schnittlinie B-B in Fig. 1) stellen kann. Die Passung darf aber keinesfalls so eng sein, dass die Bewegung des Stiftes 4 in seiner Längsrichtung senkrecht zur zu prüfenden Oberfläche behindert wird. Gegebenfalls kann der Hartmetallstift 4 zumindest teilweise auch einen eckigen Querschnitt oder andere geometrische Formen aufweisen, beispielsweise viereckig, sechseckig oder dergleichen.

Auf den Hartmetallstift 4 ist ein ringförmiger Anschlag 5 mit deutlich größerem Querschnitt als des Stiftes 4 befestigt, etwa festgeklebt, der die Endlage des Stiftes 4 zur zu prüfenden Oberfläche hin bestimmt. Dieser Stift 4 weist an seinem unteren Ende eine Spitze 5 mit 30°-Spitzenwinkel auf, die sich bei der Prüfung in eine Beschichtung der zu prüfenden Oberfläche eingräbt. Auf dem Anschlag 5 sitzt eine Druckfeder 6 auf, die den Stift 4 umgibt. Der Hartmetallstift 4 ist so bemessen, dass die ihn mit Spiel umgebende Druckfeder 6 gerade noch durch die Gewindebohrung 11 passt.

In die Gewindebohrung 11 ist von oben her eine Madenschraube 7 eingeschraubt, deren Außendurchmesser größer ist als der der Druckfeder bzw. Schraubenfeder 6. Wenn die Madenschraube 7 in die Gewindebohrung 11 so weit eingeschraubt ist, dass sie unten aus dieser heraussteht, sitzt sie somit auf der Oberseite der Druckfeder 6 auf und belastet diese mehr oder weniger, je nachdem, wie tief sie in die Gewindebohrung 11 eingeschraubt ist. Die Madenschraube 7 weist eine durchgehende Zentralbohrung 15 auf, in die der Hartmetallstift 4 hineinragt und in der er zusätzlich mit Spiel geführt wird.

An der Oberseite oder der Seitenflanke der Madenschraube 7, gegebenenfalls auch an der Oberseite des Oberteils 8, kann eine oder jeweils eine Markierung angebracht sein, die anzeigt, wie tief die Madenschraube 7 in die Gewindebohrung 11 eingeschraubt ist, und damit die Vorspannung, die auf die Druckfeder 6 aufgebracht ist.

So ist es möglich, das Gerät durch Wahl der Vorspannung an die jeweilige Beschichtung und das jeweilige Substrat der zu prüfenden Oberfläche anzupassen. Es ist zudem möglich, auf jeden der Stifte 4 eine unterschiedliche Vorspannung - durch entsprechende Einstellung der Tiefe, in die die jeweilige Madenschraube 7 eingeschraubt ist - aufzubringen und dadurch auch nach innen oder außen gewölbte Flächen zu prüfen, denn jede Spitze 12 liegt dann mit dem gleichen Druck auf der zu prüfenden Oberfläche auf.

Es kann auch die Weite des Rasters gewählt werden, das in die zu prüfende Oberfläche eingebracht wird, indem man einzelne Stifte 4 herausnimmt oder einsetzt, und zwar durch einfaches Aus- oder Einschrauben der jeweiligen Madenschraube 7; wenn diese herausgeschraubt oder eingeschraubt wird, kann nämlich der zugehörige Stift 4 mit der Druckfeder 6 durch die Gewindebohrung 11 einfach herausgehoben oder eingesetzt werden. Auch ein sehr kleines Raster, etwa 1 mm Schnittabstand, kann einfach gewählt werden, da die benachbarten Stifte 4 in Bewegungsrichtung des Geräts versetzt sind; so ist mit dem gezeigten Gerät ein Schnittabstand von 1 mm möglich, obwohl die Madenschraube einen Durchmesser von 5 mm hat. Auch ist es möglich, die relative Anordnung der Stifte 4 durch verschiedene Anordnung und Anzahl der Bohrungen 10 zu verändern, um unter geringem Aufwand verschieden beabstandete Gitterschnittlinien, beispielsweise 1, 2 oder 3 mm, zu erzeugen. Hinter den Madenschrauben 7 ist an der Oberseite des Messerkopfes 2 eine quer verlaufende Rippe ausgebildet, um die Madenschrauben 7 beim Ablegen des Geräts zu schützen.

Sollte ein Stift 4 beschädigt werden, so ist es möglich, ihn einfach auf die genannte Weise auszutauschen. Bei dem eingangs genannten, bekannten Gerät müsste in diesem Fall dagegen der gesamte Messerkörper gedreht oder ausgetauscht werden. Der Austausch des Messerkörpers wäre auch bei Ändern des Rasters erforderlich. So ist das erfindungsgemäße Gerät, obwohl es grundsätzlich mehr Teile verwendet als das bekannte Gerät, doch wesentlich einfacher, vielfältiger und, was besonders wesentlich ist, billiger. Die Stifte 4 können auch nachgeschliffen werden; in diesem Fall ist es allerdings notwendig, den Abstand zwischen der Spitze 12 und dem Anschlag 5 beizubehalten. Dies wäre durch Abschleifen oder Versetzen des Anschlages 5 oder durch Beilagen möglich, die anfangs zwischen dem Unterteil 9 und dem Anschlag 5 eingesetzt und nach jedem Nachschleifen Stück für Stück weggelassen werden.

Bei den Führungsrädern 3 tritt kein Verschleiß auf.

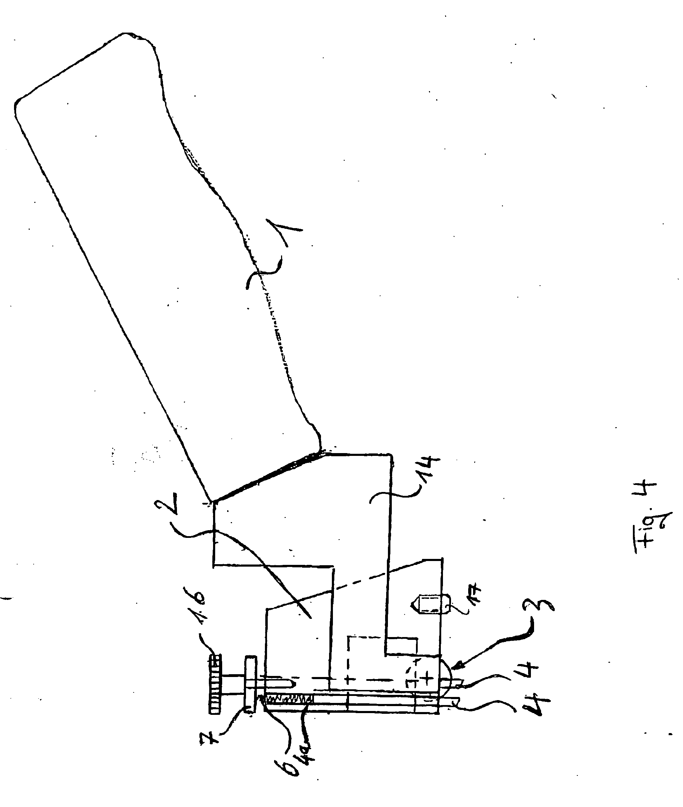

In Fig. 4 ist in einem Schnitt das Gerät gemäß einer weiteren Ausführungsform als Seitenansicht dargestellt. Gleiche und gleichbedeutende Bauteile sind mit gleichen Bezugszeichen versehen.

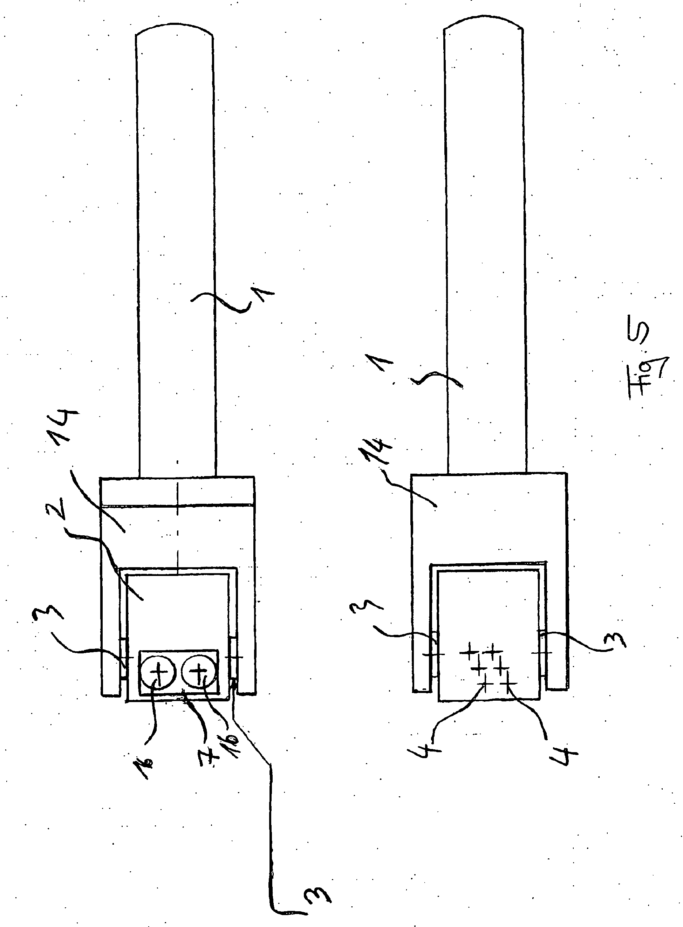

Dieser Darstellung ist - auch im Vergleich mit der in Fig. 5 von oben und von unten dargestellten Draufsicht des Gerätes der weiteren Ausführungsform - zu entnehmen, dass das Kopfteil 14 rahmenartig ausgebildet ist und zwischen zwei Armen den Messerkopf 2 aufweist. Der Messerkopf ist wiederum beiderseits mit zwei Führungsrollen 3 verbunden, die zwischen den Armen des Kopfteiles und dem Messerkopf 2 , vorzugsweise mit einer Achse drehbar, angeordnet sind.

Ebenso gibt es eine Andrückplatte 7, die die Funktion eines Auflagers übernimmt, um eine Druckfeder 6 dagegen abstützen zu lassen. Die Druckfeder 6 ist mit ihrem weiteren Ende an einem stirnseitigen Ende 4a des Hartmetallstiftes 4 gestützt. Dies findet vorzugsweise innerhalb der Bohrung, in welcher der Hartmetallstift 4 angeordnet ist, statt.

Wiederum liegt eine Vielzahl von Hartmetallstiften 4 vor.

Ebenso ist gemäß Bezugszeichen 17 ein Gleitbolzen hinter den Führungsrädern vorgesehen, der auch mehrfach angeordnet sein kann. Dieser Gleitbolzen dient zur gleitenden Bewegung des Messerkopfes auf einer zu schneidenden Unterlage.

Jedem einzelnen Hartmetallstift 4 oder mehreren Hartmetallstiften 4 zusammen ist eine oberseitig angeordnete Rändelschraube 16 zugeordnet, die es ermöglicht, die Andrückplatte 7 durch Verdrehen zu verstellen und somit die Druckfeder unterschiedlich mit Kraft zu beaufschlagen.

Der Fig. 5 ist deutlich zu entnehmen, dass die Andrückplatte größer als die Grundfläche einer Rändelschraube 16 ist. Hierdurch kann die Andrückplatte mittels in dem Fall zwei Rändelschrauben verstellt werden und zeitgleich auf mehrere Hartmetallstifte 4 wirken.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: Handgriff
- 2: Messerkopf
- 3: Führungsräder
- 4: Hartmetallstift
- 4a: Stirnseite
- 5: Anschlag
- 6: Druckfeder
- 7: Madenschraube/Andrückplatte
- 8: Oberteil
- 9: Unterteil
- 10: Durchgangsbohrung
- 11: Gewindebohrung
- 12: Spitze
- 13: Achse
- 14: Kopfteil
- 15: Zentralbohrung
- 16: Rändelschraube
- 17: Gleitbolzen

## Patentansprüche

1. Gitterschnitt-Prüfgerät mit
- einem gegen eine Beschichtung andrückbaren Messerkopf (2) mit mehreren Schneidklingen (4) und
- einem bevorzugt drehbar daran angebrachten Handgriff (1).
**dadurch gekennzeichnet, dass**
- die Schneidklingen (4) einzeln gegen die Beschichtung hin mit mindestens einer Feder (6) abgefedert sind, und
- beiderseits des Messerkopfes (2) je ein Führungskörper (3) angeordnet ist, der auf der Beschichtung aufsitzt und den Messerkopf (2) in einem bestimmten Abstand von der Beschichtung führt.

2. Gitterschnitt-Prüfgerät nach Anspruch 1,
**dadurch gekennzeichnet, dass**
der Führungskörper von einer Führungsrolle (3) gebildet ist.

3. Gitterschnitt-Prüfgerät nach einem der Ansprüche 1 oder 2,
**dadurch gekennzeichnet, dass**
jede der Schneidklingen von einem drehbar im Messerkopf (2) sitzenden, angespitzten Stift oder Hartmetallstift (4) gebildet ist.

4. Gitterschnitt-Prüfgerät nach Anspruch 3,
**dadurch gekennzeichnet, dass**
um jeden Stift oder Hartmetallstift (4) herum eine Spiral-Druckfeder (6) angeordnet ist, die sich einerseits auf einem Bund (5) des Stiftes oder Hartmetallstifts (4) und andererseits auf einem Auflager (7) des Messerkopfes (2) abstützt.

5. Gitterschnitt-Prüfgerät nach Anspruch 3,
**dadurch gekennzeichnet, dass**
ein stirnseitiges Ende eines jeden Stiftes oder Hartmetallstiftes (4) mit einer Spiral-Druckfeder (6) in Kontakt steht, die sich einerseits auf der Stirnseite (4a) des Stiftes oder Hartmetallstiftes (4) und andererseits auf einem Auflager (7) abstützt.

6. Gitterschnitt-Prüfgerät nach einem der Ansprüche 3 bis 5,
**dadurch gekennzeichnet, dass**
der Stift oder Hartmetallstift (4) einen runden oder zumindest teilweise eckigen Querschnitt aufweist.

7. Gitterschnitt-Prüfgerät nach einem der Ansprüche 4 bis 6,
**dadurch gekennzeichnet, dass**
der Stift oder Hartmetallstift (4) in einer Bohrung (10) des Messerkopfes (2) geführt ist, auf deren Einlass der Bund (5) als Endanschlag aufsitzt.

8. Gitterschnitt-Prüfgerät nach Anspruch 7,
**dadurch gekennzeichnet, dass**
der Abstand des Auflagers (7) vom Einlass der Bohrung (10) einstellbar ist.

9. Gitterschnitt-Prüfgerät nach Anspruch 7,
**dadurch gekennzeichnet,dass**
das Auflager von einer Schraubenspindel, bevorzugt einer Madenschraube (7), gebildet ist, die zur Bohrung (10) koaxial verläuft.

10. Gitterschnitt-Prüfgerät nach Anspruch 9,
**dadurch gekennzeichnet, dass**
die Schraubenspindel bzw. Madenschraube (7) und/oder die benachbarte Seite des Messerkopfes (2) markiert ist bzw. sind, um die Kraft der Druckfeder (6) einzustellen.

11. Gitterschnitt-Prüfgerät nach mindestens einem der Ansprüche 1 bis 10,
**dadurch gekennzeichnet, dass**
die geometrische Anordnung mehrerer Bohrungen (10) im Messerkopf (2) zumindest teilweise senkrecht und/oder schräg zur Schnittrichtung (Bewegungsrichtung des Geräts) erfolgt.

12. Gitterschnitt-Prüfgerät nach mindestens einem der Ansprüche 1 bis 11,
**dadurch gekennzeichnet, dass**
mindestens ein Teil der Stifte oder Hartmetallstifte (4) zueinander in Schnittrichtung (Bewegungsrichtung des Geräts) versetzt angeordnet ist.

13. Gitterschnitt-Prüfgerät nach einem der Ansprüche 1 bis 12,
**dadurch gekennzeichnet, dass**
einzelne Stifte oder Hartmetallstifte (4) unwirksam gemacht oder ausgebaut werden können.

## Claims

1. Cross-cut test device with
- a cutter head (2) that is pressable against a coating and has several cutting edges (4) and
preferred handle rotably(1) attached to it
**characterised in that**
- the cutting edges (4) are individually cushioned towards the coating by at least one spring (6), and
a guiding device (3) is each located on both sides of the cutter head (2), which is rested on the coating and guides the cutter head (2) in a specific distance to the coating.

2. Cross-cut test device according to claim 1,
**characterised in that**
the guiding device is made out of a guiding roll (3).

3. Cross-cut test device according to one of the claims 1 or 2,
**characterised in that**
each cutting edge is made out of a bolt or a carbide bolt which is fixed pivotable to the cutter head (2).

4. Cross-cut test device according to claim 3,
**characterised in that**
a spiral pressure spring (6) is placed around each bolt or carbide bolt (4), which on the one hand is held up by a flange (5) of the bolt or carbide bolt (4) and on the other hand is held up by a rest (7) of the cutter head (2).

5. Cross-cut test device according to claim 3,
**characterised in that**
one front end of any bolt or any carbide bolt (4) is in contact with a spiral pressure spring (6) which on the one hand is held up by the front end (4a) of the bolt or carbide bolt (4) and on the other hand is held up by a rest (7).

6. Cross-cut test device according to one of the claims 3 to 5,
**characterised in that**
the bolt or carbide bolt (4) shows a round or at least partially angularly cross section

7. Cross-cut test device according to one of the claims 4 to 6,
**characterised in that**
the bolt or carbide bolt (4) is guided in a borehole (10) of the cutter head (2), on the inlet of which the flange (5) is rested as an end stop.

8. Cross-cut test device according to claim 7,
**characterised in that**
the distance of the rest (7) to the inlet of the borehole (10) is adjustable

9. Cross-cut test device according to claim 7,
**characterised in that**
the rest is made out of a screw spindle, preferably a headless screw (7), which runs coaxially with respect to the borehole (10).

10. Cross-cut test device according to claim 9,
**characterised in that**
the screw spindle respectively the headless screw (7) and/or the adjoining site of the cutter head (2) is respectively are marked, to adjust the power of the pressure spring (6).

11. Cross-cut test device according to at least one of the claims 1 to 10,
**characterised in that**
the geometric arrangement of several boreholes (10) in the cutter head (2) is carried out at least partially vertically and/or inclined with respect to the cutting direction (direction of movement of the test device).

12. Cross-cut test device according to at least one of the claims 1 to 11,
**characterised in that**
at least a part of the bolts or the carbide bolts (4) is arranged offset to each other in the cutting direction (direction of movement of the test device).

13. Cross-cut test device according to one of the claims 1 to 12,
**characterised in that**
single bolts or carbide bolts (4) can be rendered ineffective or dismantled.

## Revendications

1. Appareil d' essai de quadrillage avec
- une tête de couteau (2) avec plusieurs lames tranchantes qui permet d'être appuyer contre un revêtement et
- une poignée (1) étant attachée à celui-ci de façon préferée pivotante
**caractérisé en ce que**
- les lames tranchantes (4) sont chacune amorties individuellement contre le revêtement avec au moins un ressort (6), et
- un dispositif de guidage (3) chaque mis en place de chaque côté de la tête de couteau (2), celui-ci restant sur le revêtement et gardant la tête de couteau (2) à une distance spécifique au-dessus du revêtement.

2. Appareil d' essai de quadrillage selon la revendication 1,
**caractérisé en ce que**
le dispositif de guidage est fait à partir d'un rouleau de guidage (3).

3. Appareil d' essai de quadrillage selon une des revendications 1 ou 2,
**caractérisé en ce que**
chaque lame est faite à partir d'une cheville ou une cheville de métal dur qui est tenue pivotantement dans la tête du couteau (2).

4. Appareil pour essai de quadrillage selon revendication 3,
**caractérisé en ce que**
un ressort à pression en spiral (6) est placé autour de chaque cheville ou cheville de métal dur (4), celui-ci est appuyé d'un côté contre un épaulement (5) de la cheville ou la cheville de métal dur (4) et de l'autre côté contre un appui (7) sur la tête du couteau.

5. Appareil d' essai de quadrillage selon la revendication 3,
**caractérisé en ce que**
un front de chaque cheville ou de chaque cheville de métal dur (4) est en contact avec un ressort à pression en spiral (6), celui-ci est appuyé d'un côté contre un front (4a) de la cheville ou cheville de métal dur (4) et de l'autre côté contre un appui (7).

6. Appareil pour essai de quadrillage selon un des revendications 3 a 5,
**caractérisé en** ceque
la cheville ou la cheville de métal dur (4) participe d' une coupe transversale ronde ou au moins partiellement carrée.

7. Appareil d' essai de quadrillage selon un des revendications 4 à 6,
**caractérisé en ce que**
la cheville ou la cheville de métal dur (4) est guidée dans un trou percé (10) dans la tête du couteau (2), sur l'entrée de celui-ci l'épaulement (5) est attaché comme un arrêt final.

8. Appareil d' essai de quadrillage selon la revendication 7,
**caractérisé en ce que**
la distance de l'appui (7) à l'ouverture du trou (10) est ajustable.

9. Appareil d' essai de quadrillage selon revendication 7,
**caractérisé en ce que**
l'appui est fait à partir d'une broche à vis, de préférence une vis sans tête (7), qui est disposée coaxialement au trou percé (10).

10. Appareil d'essai de quadrillage selon la revendication 9,
**caractérisé en ce que**
la broche à vis ou bien la vis sans tête (7) et/ou le côté adjacent de la tête de couteau (2) est ou bien sont marqués, afin d'ajuster la force du ressort à pression (6).

11. Appareil d' essai de quadrillage selon au moins un des revendications 1 a 10,
**caractérisé en ce que**
la disposition géométrique de plusieurs trous percés (10) dans la tête du couteau (2) s'effectue au moins partiellement verticalement et/ou de façon inclinée par rapport à la direction de coupe (direction de mouvement de l'appareil).

12. Appareil d' essai de quadrillage selon au moins une des revendications 1 a 11,
**caractérisé en ce que**
au moins une partie des chevilles ou des chevilles de métal dur (4) est disposée en étant décalée axialement l'une par rapport à l'autre dans le sens de la découpe (direction de mouvement de l'appareil).

13. Appareil d' essai de quadrillage selon une des revendications 1 a 12,
**caractérisé en ce que**
des chevilles individuelles ou des chevilles de métal dur (4) peuvent être rendues inopérantes ou démontées.
